(19) Europäisches Patentamt
European Patent Office
Office européen des brevets

(11) **EP 4 706 500 A1**

(12) **EUROPEAN PATENT APPLICATION**

(43) Date of publication:
**11.03.2026 Bulletin 2026/11**

(21) Application number: **25198740.0**

(22) Date of filing: **28.08.2025**

(51) International Patent Classification (IPC):
**A61B 3/10** *(2006.01)* **A61B 5/00** *(2006.01)*

(52) Cooperative Patent Classification (CPC):
**A61B 3/10; A61B 3/101;** A61B 5/01; A61B 5/4833;
A61B 5/6803; A61B 5/7275

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **10.09.2024 FI 20246105**

(71) Applicant: **IXI Eyewear Oy**
**02150 Espoo (FI)**

(72) Inventors:
- **Virtanen, Juha**
**00560 Helsinki (FI)**
- **Pitkänen, Ari**
**01200 Vantaa (FI)**
- **Wederhorn, Roosa**
**02110 Espoo (FI)**

(74) Representative: **Moosedog Oy**
**Kurjenmäenkatu 10 B 49**
**20700 Turku (FI)**

(54) **METHOD AND OPTICAL APPARATUS FOR MONITORING USE OF EYE DROPS**

(57) Disclosed is method for monitoring use of eye drops in user's eye (300). The method comprising measuring temperature of ocular surface (OS) of user's eye, at first plurality of time instants within first time duration (FTD), prior to optical apparatus (OA) (200) not being in use for predefined time period; generating first temperature trend (FTT), for FTD; measuring temperature of OS of user's eye, at second plurality of time instants within second time duration (STD), subsequent to OA being in use, wherein OA is brought to use after OA not being in use for predefined time period; generating second temperature trend (STT), for STD; determining change in temperature trend between FTT and STT; determining if change in temperature trend is greater than predefined value; and when it is determined that change in temperature trend is greater than predefined value, deeming use of the eye drops in the user's eyes.

FIG. 2

EP 4 706 500 A1

## Description

TECHNICAL FIELD

The present disclosure relates to methods for monitoring use of eye drops in user's eye. Moreover, the present disclosure relates to optical apparatuses.

BACKGROUND

Recently, instillation of eye drops is a critical aspect of treatment for various ocular conditions such as glaucoma, dry eye syndrome and infections. However, a significant portion of patients struggle with proper application of the eye drops. The misapplication leads to inadequate treatment, exacerbation of the condition and potential complications. For example, nearly one-third of subjects who believed they had successfully applied eye drops actually missed their eyes. Moreover, only about 9% of glaucoma patients were able to properly self-administer their eye drops.

Existing solutions to address the problem of monitoring eye drop application involve a use of fluorescent dyes added to the eye drops, which can be detected by a photo detector integrated into eyeglass frames. The system uses an LED to excite the fluorescent agent, indicating whether the eye drops have been applied. Another existing solution includes using electrical drop counters attached to eye drop bottles to count the number of drops dispensed. Additionally, more advanced solutions involve cameras to ensure the eye drop is correctly applied to the eye. While the aforementioned solutions provide some level of monitoring, however, they have significant limitations, such as requiring specific modifications to eye drop formulations or relying on complex and often expensive equipment.

Despite the above mentioned advancements, existing solutions still fall short in several areas. Fluorescent dye-based systems may require changes to eye drop formulations that are not always feasible or desirable. Electrical drop counters only indicate the number of drops dispensed, not whether they have been correctly applied to the eye. Camera-based systems, while more accurate, can be intrusive and cumbersome for everyday use. Furthermore, the aforementioned solutions may not effectively account for the variability in individual application techniques and physiological differences among patients.

Therefore, in light of the foregoing discussion, there exists a need to overcome the aforementioned drawbacks.

SUMMARY

The aim of the present disclosure is to provide a method and an optical apparatus to ensure a correct and regular use of eye drops by a user. The aim of the present disclosure is achieved by a method and an optical apparatus for monitoring a use of eye drops in a user's eye as defined in the appended independent claims to which reference is made to. Advantageous features are set out in the appended dependent claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

BRIEF DESCRIPTION OF THE DRAWINGS

FIG. 1 is an illustration of a flowchart for depicting steps of a method for monitoring a use of eye drops in a user's eye, in accordance with an embodiment of the present disclosure;

FIG. 2 is a schematic illustration of an optical apparatus, in accordance with an embodiment of the present disclosure;

FIG. 3 is a schematic illustration of a user's eye when an optical apparatus is in use, in accordance with an embodiment of the present disclosure; and

FIG. 4 is a graphical representation of a first temperature trend and a second temperature trend, in accordance with an embodiment of the present disclosure.

FIGs. 5A-C are graphical representations of first temperature trend and second temperature trend for first time duration and the second time duration, respectively, in accordance with an embodiment of the present disclosure.

DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides a method for monitoring a use of eye drops in a user's eye, wherein the method comprising:

measuring a temperature of an ocular surface of the user's eye, using at least one temperature sensor in an optical apparatus worn by a user, at a first plurality of time instants within a first time duration, prior to the optical apparatus not being in use for a predefined time period, wherein the first time duration corresponds to a period when the optical apparatus is worn by the user before the optical apparatus is taken off;

wherein the at least one temperature sensor is at least one thermopile IR sensor configured to measure infrared emission between a range of 8 to 14 micrometers;

generating a first temperature trend of the ocular surface of the user's eye, for the first time duration, based on the measured temperature at the first plurality of time instants within the first time duration, using at least one processor in the optical apparatus communicably coupled to the at least one temperature sensor, wherein a normal ocular temperature of the user's eye is in a range of 32°C and 36°C;

wherein the predefined time period of the optical apparatus not being in use is in a range of 30 seconds to 5 minutes, and wherein the first time duration and the second time duration are in a range of 5 minutes to 15 minutes;

measuring the temperature of the ocular surface of the user's eye, using the at least one temperature sensor, at a second plurality of time instants within a second time duration, subsequent to the optical apparatus being in use, wherein the optical apparatus is brought to use after the optical apparatus not being in use for the predefined time period ,wherein the optical apparatus is brought to use after the optical apparatus not being in use for the predefined time period, wherein the second time duration corresponds to a period when the optical apparatus is worn by the user after the optical apparatus is put back on;

generating a second temperature trend of the ocular surface of the user's eye, for the second time duration, based on the measured temperature at the second plurality of time instants within the second time duration, using the at least one processor;

determining a change in temperature trend between the first temperature trend and the second temperature trend of the ocular surface of the user's eye, using the at least one processor;

determining if the change in temperature trend is greater than a predefined value, using the at least one processor; and

when it is determined that the change in temperature trend is greater than the predefined value, deeming the use of the eye drops in the user's eyes during the predefined time period of the optical apparatus not being in use.

The present disclosure provides an aforementioned method that is able monitor the use of eye drops in a user's eye accurately and continuously for the time period. Moreover, the method is able to detect even subtle changes in ocular surface temperature that are indicative of the eye drop application, by comparing the first and the second temperature trends. Furthermore, the method eliminates the need of self-reporting or manual monitoring by the user, providing an automated and objective way to monitor the use of eye drops. The use of at least one temperature sensor and at least one processor allows for continuous and consistent data collection, reducing the possibility of human error.

In a second aspect, the present disclosure provides an optical apparatus comprising:

a frame;

at least one temperature sensor arranged on the frame, wherein when the optical apparatus is in use, the at least one temperature sensor faces a user's eye, wherein the at least one temperature sensor is at least one thermopile IR sensor configured to measure infrared emission between a range of 8 to 14 micrometers; and

at least one processor coupled to the at least one temperature sensor, wherein the at least one processor is configured to:

control the at least one temperature sensor to measure a temperature of an ocular surface of the user's eye, at a first plurality of time instants within a first time duration, prior to the optical apparatus not being in use for a predefined time period, wherein the first time duration corresponds to a period when the optical apparatus is worn by the user before the optical apparatus is taken off;

generate a first temperature trend of the ocular surface of the user's eye, for the first time duration, based on the measured temperature at the first plurality of time instants within the first time duration, wherein a normal ocular temperature of the user's eye is in a range of 32°C and 36°C;

wherein the predefined time period of the optical apparatus not being in use is in a range of 30 seconds to 5 minutes, and wherein the first time duration and the second time duration are in a range of 5 minutes to 15 minutes;

control the at least one temperature sensor to measure the temperature of the ocular surface of the user's eye, at a second plurality of time instants within a second time duration, subsequent to the optical apparatus being in use, wherein the optical apparatus is brought to use after the optical apparatus not being in use for the predefined time period;

generate a second temperature trend of the ocular surface of the user's eye, for the second time duration, based on the measured temperature at the second plurality of time instants within the second time duration, wherein the second time duration corresponds to a period when the optical apparatus is worn by the user after the optical apparatus is put back on;

determine a change in temperature trend between the first temperature trend and the second temperature trend of the ocular surface of the user's eye;

determine if the change in temperature trend is greater than a predefined value; and

when it is determined that the change in temperature trend is greater than the predefined value, deem the use of the eye drops in the user's eyes during the time period of the optical apparatus not being in use.

The present disclosure provides an aforementioned optical apparatus that is able to monitor the temperature of the ocular surface of the user's eye effectively and continuously for the time period. Moreover, the at least one temperature sensor in the optical apparatus provides a simple and convenient way for estimating the temperature of the ocular surface of the user's eye in a non-contact and easy to implement manner. Furthermore, the optical apparatus eliminates the need for user self-reporting or manual monitoring, providing an automated and objective way to monitor the use of eye drops. Furthermore, use of the at least one processor allows for continuous and consistent data collection, reducing the possibility of human error.

Throughout the present disclosure, the term *"monitoring"* refers to a process of continuously detecting and tracking the use of the eye drops in the user's eye. Notably, monitoring the use of eye drops in the user's eye ensures adherence to a prescribed eye drops usage regime, which is crucial for treating eye conditions and ocular diseases such as glaucoma, dry eye syndrome and the like. Moreover, a significant number of users fails to properly self-administer the eye drops, thus monitoring the eye drop usage facilitates in promoting compliance and optimizing treatment outcomes. Throughout the present disclosure, the term *"eye drops"* refers to a liquid medicament designed to be applied to the user's eye. Typically, the eye drops is used to deliver drugs or hydration to the ocular surface of the user's eye for therapeutic or preventive purposes.

Throughout the present disclosure, the term *"ocular surface of the user's eye"* refers to a surface around the user's eye that comprises at least one of: a corneal surface of the user's eye, a surrounding skin surface of the corneal surface, an eyelid surface. Herein, the term *"temperature"* refers to an internal temperature of the ocular surface of the user's eye. The ocular temperature of the user's eye being in a range of 32°C and 36°C is considered to be of a normal level. Notably, measuring the temperature of the ocular surface of the user's eye is crucial for monitoring the use of the eye drops in the user's eye, as significant changes in the temperature of the ocular surface is indicative of the use of the eye drops in the user's eye. It will be appreciated that the temperature of the ocular surface of the user's eye is measured by determining an average of measured temperature of at least one point on the ocular surface of the user's eye. Throughout the present disclosure, the term *"temperature sensor"* refers to a sensing device that is able to sense a temperature of any surface. Notably, the at least one temperature sensor is used for measuring the temperature of the ocular surface of the user's eye at the first plurality of time instants within the first time duration which implies that the temperature of the ocular surface of the user's eye is measured at each time instant amongst the first plurality of time instants within the first time duration. For example, the at least one temperature sensor is at least one of: thermocouples, resistance temperature detectors, thermistors, or infrared sensors. In an implementation, the at least one temperature sensor comprises a plurality of temperature sensors which enables to cover a broader area of the ocular surface of the user's eye.

The at least one temperature sensor is at least one thermopile IR sensor. In this regard, the term *"thermopile IR sensor"* refers to a non-contact temperature sensor that measures infrared radiation (heat) emitted from the eye of the user to determine the temperature of the ocular surface of user's eye. It will be appreciated that the at least one thermopile IR sensor is well-known in the art. Typically, the at least one thermopile IR sensor operates based on thermoelectric effect. Notably, the *"at least one thermopile IR sensor"* refers to *"a thermopile IR sensor"* in some implementations, and *"a plurality of thermopile IR sensors"* in other implementations. Notably, the at least one thermopile IR sensor is able to measure the infrared emission between a range of 8 to 14 micrometers. A technical effect is that the at least one temperature sensor is accurate, fast, cost effective and reliable to detect minute changes in the temperature of the ocular surface of the user's eye.

Optionally, a sensing area of the at least one temperature sensor is aligned with a horizontal axis passing through the user's eye. In this regard, the term *"sensing area"* refers to a particular area within the ocular surface of the user's eye in which the at least one temperature sensor is able to measure the temperature of the ocular surface of the user's eye. Optionally, the sensing area of the at least one temperature sensor is in a shape of a circle, an oval, or any polygonal shape. Notably, the sensing area defines the boundaries on the ocular surface of the user's eye within which the at least one temperature sensor is able to operate. It will be appreciated that the sensing area for each temperature sensor amongst the at least one temperature sensor is different. Throughout the present disclosure, the term *"horizontal axis"* refers to an imaginary straight line that extends horizontally through a center of the user's eye. Optionally, the horizontal axis act as a reference line for placement of the at least one temperature sensor within the optical apparatus. As a result, the at least one temperature sensor is positioned within the optical apparatus in such a way that, the at least one temperature sensor is facing the user's eye for the accurate measurement of the temperature of the ocular surface of the user's eye. Moreover, when the user wears the optical apparatus, the position of the at least one temperature sensor ensures that the at least one temperature sensor is aligned with the horizontal axis of the user's eye. A technical effect of the alignment of the sensing area with the horizontal axis of the user's eye ensures that the measured temperature is accurate and reflective of the temperature of the ocular surface of the user's eye.

Throughout the present disclosure, the term *"optical apparatus"* refers to an arrangement of electrical and/or mechanical components that is worn around eyes of the user. Optionally, the optical apparatus (practically, a smart eyewear) is employed, for example, to correct a vision disorder, including but not limited to, myopia, hypermetropia, astigmatism, presbyopia and the like, such as, by enabling appropriate refraction, reflection, diffraction or transmission of light towards the eyes of the user wearing the optical apparatus. Beneficially, the optical apparatus is used to provide protection to the eyes of the user from ambient conditions such as sunlight, ultraviolet radiation, blue light associated with computing devices, dust, chemical fumes, and such like. Moreover, the optical apparatus enables display of information to the eyes of the user. Throughout the present disclosure, the term *"first time duration"* refers to a predefined duration of time for which the temperature of the ocular surface of the user's eye is to be tracked prior to the optical apparatus not being in use. In this regard, the first time duration is the time duration before the optical apparatus is taken off by the user. Throughout the present disclosure, the term *"first plurality of time instants"* refers to multiple different instances of time spread across the entire period of the first time duration. Subsequently, measuring the temperature of the ocular surface of the user's eye at the first plurality of time instants enables to track the temperature of the ocular surface of the user's eye over the entire period of the first time duration. Moreover, measuring the temperature of the ocular surface of the user's eye at the first plurality of time instants within the first time duration, enables to collect the data points by the at least one temperature sensor required for generating the first temperature trend.

Throughout the present disclosure, the term *"predefined time period"* refers to a specific time period during which the optical apparatus is not being in use (i.e., not worn by the user). Typically, a duration of the predefined time period can vary, depending on the user habits or specific monitoring protocols. The predefined time period of the optical apparatus not being in use is in a range of 30 seconds to 5 minutes, and wherein the first time duration and the second time duration are in a range of 5 minutes to 15 minutes. In this regard, the predefined time period of the optical apparatus not being in use in in range of 30 seconds to 5 minutes. Optionally, the predefined time period of the optical apparatus may be in the range of 30 seconds, 1 min, 2 min, 3 min or 4 min up to 1, 2, 3, 4 or 5 min. Optionally, the first time duration and the second time duration may be in the range of 5, 7, 9, 11 or 13 min up to 7, 9, 11, 13 or 15 min. Notably, the predefined time period is measured to ensure whether the predefined time period fall within the range of 30 seconds to 5 minutes. If the predefined time period does not fall within the range of 30 seconds to 5 minutes, then the predefined time period in not considered as a validate time period for the use of eye drops. Optionally, the first time duration is different from the second time duration. Notably, the first time duration and the second time duration needs to be long enough to measure sufficient data points of the temperature of the ocular surface of the user's eye for generating the first temperature trend and the second temperature trend. Moreover, the predefined time period needs to be long enough to create an effective distinction between the first temperature trend and the second temperature trend. A technical effect is that the predefined time period, the first time duration and the second time duration are selected to be long enough to facilitate effective generation of the first temperature trend and the second temperature trend.

Throughout the present disclosure, the term *"first temperature trend"* refers to a pattern or trend depicting changes in the temperature of the ocular surface of the user's eye, measured at the first plurality of time instants within the first time duration. Typically, the first temperature trend is generated for the first time duration prior to the optical apparatus not being in use, based on the measured temperature at the first plurality of time instants during the first time duration. Moreover, the measured temperature of the ocular surface at the first plurality of time instants within the first time duration is processed by the at least one processor of the optical apparatus to create a continuous or discrete representation of how the ocular surface temperature changes over the first time duration, in form of the first temperature trend, prior to the optical apparatus not being in use for the predefined time period. Optionally, the first temperature trend may be generated as a graph or stored as a series of data points that reflects the temperature of the ocular surface at the first plurality of time instants within the first time duration.

Throughout the present disclosure, the term *"processor"* refers to a computational element that is operable to execute instructions of the optical apparatus. Examples of the at least one processor include, but are not limited to, a microprocessor, a microcontroller, a complex instruction set computing (CISC) microprocessor, a reduced instruction set (RISC) microprocessor, a very long instruction word (VLIW) microprocessor, or any other type of processing circuit. Furthermore, the at least one processor may refer to one or more individual servers, processing devices and various elements associated with a processing device that may be shared by other processing devices. Additionally, one or more individual processors, processing devices and elements are arranged in various architectures for responding to and processing the instructions that execute the instructions of the optical apparatus. Optionally, the at least one processor is coupled to the at least one temperature sensor in a wired or wireless manner. Notably, prior to estimating the temperature of the ocular surface of the user's eye, the at least one processor is configured to receive sensor data from the at least one temperature sensor, the sensor data being indicative of the measured temperature of the first plurality of time instants. Moreover, the at least one processor analyzes the sensor data to generate the first temperature trend, representing how the temperature of the ocular surface of user's eye changes over the first time duration. Beneficially, the first temperature trend measured at the plurality of first time instants reduces the influence of transient temperature variations, leading to precise and consistent monitoring results.

Notably, the temperature of the ocular surface of the user's eye is also measured at the second plurality of time instants within the second time duration. Throughout the present disclosure, the term *"second time duration"* refers to a predefined duration of time for which the temperature of the ocular surface of the user's eye is to be tracked subsequent to the optical apparatus brought to use after the optical apparatus not being in use for the predefined time period. Notably, the measurement of the temperature within the second time duration occur after the optical apparatus is brought to use (i.e., worn again by the user) after the predefined time period during which the optical apparatus is not in use. Throughout the present disclosure, the term *"second plurality of time instants"* refers to multiple different instances of time spread across the entire period of the second time duration. Moreover, measuring the temperature of the ocular surface of the user's eye at the second plurality of time instants within the second time duration, by the at least one temperature sensor is crucial for detecting changes in the temperature of the ocular surface after the optical apparatus is brought to use again after the predefined time period. Beneficially, by measuring the temperature of the ocular surface at the second plurality of time instants within the second time duration, enough data points are collected by the at least one temperature sensor for the at least one processor for generating the second temperature trend.

Throughout the present disclosure, the term *"second temperature trend"* refers to a pattern or trend in changes in the temperature of the ocular surface of the user's eye, measured at the second plurality of time instants within the second time duration. Typically, the second temperature trend is generated for the second time duration when the optical apparatus is again worn by the user after the predefined time period, based on the measured temperature at the second plurality of time instants during the second time duration. Moreover, the measured temperature of the ocular surface at the second plurality of time instants within the second time duration is processed by the at least one processor of the optical apparatus to create a continuous or discrete representation of how the ocular surface temperature changes over the second time duration, in form of the second temperature trend after the optical apparatus is brought to use again after the predefined time period. Optionally, the second temperature trend may be generated as a graph or stored as a series of data points that reflects the temperature of the ocular surface at the second plurality of time instants within the second time duration. Notably, the at least one processor analyzes the sensor data of the at least one temperature sensor for generating the second temperature trend, representing how the temperature of the ocular surface of user's eye changes over the second time duration.

Throughout the present disclosure, the term *"change in temperature trend"* refers to a quantifiable difference in the pattern or trajectory of the temperature trend of the ocular surface of the user's eye between the first temperature trend and the second temperature trend. Notably, the change in temperature trend is determined to deem the use of the eye drops. Typically, the use of the eye drops causes a noticeable change in the temperature of the ocular surface of the user's eye due to cooling effect of the eye drops and increased evaporation rate, following the application of the eye drops. Furthermore, by comparing the temperature trends, the at least one processor is able to deem whether eye drops are used during the predefined time period when the optical apparatus is not being in use. Notably, the at least one processor compares the first and the second temperature trend to identify the change in temperature trend. Optionally, the comparison involves statistical analysis to determine the change between the first temperature trend and the second temperature trend.

Optionally, the step of determining the change in temperature trend comprises determining one of:

    a change in slope between a first slope of the first temperature trend and a second slope of the second temperature trend,
    a change in average temperature between a first average temperature of the first temperature trend and a second average temperature of the second temperature trend,
    a change in initial temperature between a first initial temperature in the first temperature trend and a second initial temperature in the second temperature trend, or

a change in given temperature between a first given temperature amongst the first temperature trend at a first given time instant amongst the first duration and a second given temperature amongst the second temperature trend at a second given time instant amongst the second time duration.

In this regard, the term *"first slope"* refers to the rate of change of the temperature of the ocular surface of the user's eye over time during the first time duration for the first temperature trend. The term *"second slope"* refers to the rate of change of the temperature of the ocular surface of the user's eye over time during the second time duration of the second temperature trend. For example, when the first temperature trend is represented as a series of temperature (T) and time (t) pairs such as $(t_1, T_1)$, $(t_2, T_2)$, .... , $(t_n, T_n)$, the first slope

$$m_1 = \frac{T_n - T_1}{t_n - t_1}$$ can be calculated using an initial and a final point in the first time duration. Similarly, the second slope can be calculated using the initial and final points in the second time duration. Herein, the phrase *"change in slope"* refers to a difference between the first slope of the first temperature trend and the second slope of the second temperature trend.

Herein, the term *"first average temperature"* refers to a mean temperature value of the ocular surface measured over the first plurality of time instants within the first time duration for the first temperature trend. Herein, the term *"second average temperature"* refers to the mean temperature value of the ocular surface measured over the second plurality of time instants within the second time duration for the second temperature trend. Herein, the phrase *"change in average temperature"* refers to a difference between the first average temperature of the ocular surface measured during the first time duration and the second average temperature of the ocular surface measured during the second time duration. Beneficially, the change in average temperature facilitates the user to observe whether there is a significant alteration in the ocular surface temperature, which may indicate the use of eye drops. The change in the average temperature can signify that the user has applied the eye drops during the predefined time period when the optical apparatus is not in use.

Herein, the term *"first initial temperature"* refers to the temperature of the ocular surface of the user's eye measured at an initial time instant amongst the first plurality of time instants using the at least one temperature sensor. Herein, the term *"second initial temperature"* refers to the temperature of the ocular surface of the user's eye measured at an initial time instant amongst the second plurality of time instants using the at least one temperature sensor. For example, suppose the at least one temperature sensor starts measuring the temperature of the ocular surface of the user's eye at time $t_0$ during the first time duration, then the measured temperature of the ocular surface at $t_0$ is the first initial temperature. Similarly, the second initial temperature can be measured. Notably, the first initial temperature in the first temperature trend serve as a baseline to compare against the second initial temperature in the second temperature trend.

Herein, the term *"first given temperature"* refers to the measured temperature of the ocular surface of the user's eye at the first given time instant amongst the first plurality of time instants within the first time duration. For example, the temperature of the ocular surface measured at the first given time instant $(t_1)$ within the first time duration is the first given temperature. Herein, the term *"second given temperature"* refers to the measured temperature of the ocular surface of the user's eye at the second given time instant amongst the second plurality of time instants within the first time duration. For example, the temperature of the ocular surface measured at the second given time instant $(t_2)$ within the second time duration is the second given temperature. Herein, the phrase *"change in given temperature"* refers to the difference between the first given temperature amongst the first temperature trend and the second given temperature amongst the second temperature trend. Furthermore, the aforementioned ways of determining the change in temperature trend are crucial for accurately identifying significant temperature variations in ocular surface of the user's eye that indicate the use of the eye drops. The change in the slope, the change in average temperature, the change in initial temperature and the change in given temperature provides comprehensive insights into the temperature variation of the ocular surface, enhancing robustness and reliability of the optical apparatus. A technical effect of aforementioned claim is that the change in the temperature trend is effectively determined. Furthermore, by offering multiple approaches to determine the change in the temperature trend, the at least one processor is able to adapt to different scenarios and user behaviors, providing reliability and robustness in determining the change in temperature trend.

Throughout the present disclosure, the term *"predefined value"* refers to a threshold value to be compared with the change in temperature trend.

Notably, the predefined value is used by the at least one processor to evaluate a significance of the change in temperature trend. It will be appreciated that the change in temperature trend being greater than the predefined value implies that the change in temperature trend is significant enough to indicate the use of the eye drops in the user's eye during the predefined time period. Moreover, the predefined value simplifies decision-making process for the at least one processor by providing a clear cut-off point to compare with the change in temperature trend. Furthermore, the predefined value is determined, based on factors such as clinical guidelines, experimental data and the like. Subsequently, the at least one processor compares the determined change in temperature trend to the predefined value for determining whether the change in temperature trend is greater than the predefined value.

Optionally, the predefined value is adjusted based on a type of the eye drops, and wherein the determined change in temperature trend is indicative of a decline when the type of the eye drops is lipid-free eye drops. In this regard, the phrase *"type of the eye drops"* refers to a specific category or formulation of the eye drops characterized by primary ingredients, purpose, composition and physical properties of the eye drops. Notably, the type of the eye drops can be lipid-free eye drops, lipid emulsions, oil-based eye drops, medicated eye drops and the like. Notably, the predefined value for determining the changes in the temperature trend may vary based on the type of the eye drops used. Notably, if the type of the eye drops is lipid-free eye drops, then the temperature of the ocular surface of the user's eye is reduced significantly due to high evaporation rate, and subsequently the change in temperature trend is indicative of the decline immediately after the application of lipid-free eye drops. However, the evaporation rate of the lipid-free eye drops is slower and the temperature of the ocular surface of the user's eye drifts back to normal. Thus, the temperature trend of the ocular surface temperature changes either positively or negatively as compared to the temperature trend of the ocular surface of the user's eye without the application of the eye drops. However, if the eye drops are lipid emulsions, then the temperature of the ocular surface of the user's eye may decrease due to lower evaporation rate, and subsequently the change in temperature trend is indicative of an average drop. It will be appreciated that the lipid content of the eye drops is unable to significantly affect the temperature trend of the user's eye. Optionally, the effect of the eye drops depends upon the condition of the user's eye. For example, if the user's eye has gone under surgery, then the effect of the eye drops in the user's eye is more significant as compared to the effect of the eye drops in user's other eye (i.e. the other eye that has not undergone surgery), regardless of the type of the eye drops. A technical effect is that an accuracy and effectiveness in comparison with the change in temperature trend is significantly improved.

Moreover, the at least one processor evaluates the change in temperature trend and subsequently, when it is determined that the change in temperature trend is greater the predefined value, the at least on processor deems that the eye drops are used by the user during the predefined time period when the optical apparatus is not being in use. Beneficially, determining when the change in temperature trend is greater than the predefined value, provides an automated method for deeming the use of the eye drops without the need for manual input or observation.

Optionally, the method further comprises storing data related to the deemed use of the eye drops in the user's eye during the predefined time period of the optical apparatus not being in use in a database. In this regard, the term *"database"* refers to an organized collection of data that is stored and accessed electronically. Typically, the database is designed to efficiently store, retrieve, manage, and manipulate data in various formats such as tables, records, indexes, queries, fields, reports and the like. Notably, the database can be of various types such as relational database, NoSQL database, In-Memory database, cloud database, distributed database and the like. Moreover, the database stores the data related to the deemed use of the eye drops, particularly during the predetermined time period when the optical apparatus is not being used. Optionally, the stored data may include timestamps, temperature trends, change in the temperature trend, determination of the application of the eye drops and the like. Furthermore, storage of the data related to the deemed use of the eye drops, over time facilitates understanding the patterns and trends in usage of the eye drops. The storage of the data in the database allows for easy access and retrieval by the user for further evaluation and management. Furthermore, using the database ensures that sensitive data of the user is stored securely and protects privacy of the user. A technical effect of storing the data related to the deemed use of the eye drops over time is that deeper insights and understanding about long-term adherence patterns and effectiveness of the usage of the eye drops are determined.

Optionally, the method further comprises:

generating a feedback for the user, based on the determined change in temperature trend, using the at least one processor; and
sending the feedback as an alert message to a user device communicably coupled to the at least one processor.

In this regard, the term *"feedback"* refers to an information or response generated by the at least one processor based on the analysis of the change in temperature trend. Typically, the feedback is intended to inform the user about results of the monitoring process regarding the use of the eye drops. The term *"user device"* refers to an electronic device associated with (or used by) the user that is capable of enabling the user to perform specific tasks. Examples of the user device include, but are not limited to, cellular phones, personal digital assistants (PDAs), handheld devices, wireless modems, laptop computers, personal computers, etc. Herein, the term *"alert message"* refers to a notification or a communication sent to the user device to inform the user about the feedback based on the determined change in temperature trend, generated by the at least one processer. Typically, the alert message serves to alert the user about the generated feedback. Beneficially, the alert message ensures that the user receives timely updates and notification about the generated feedback. Optionally, the alert message may include text, graphics and the like which is sent to the user device via a wireless or a wired network. Optionally, the user device receives the alert message and notifies the user through visual cues, auditory cues and the like. A technical effect of the aforementioned claim is that the alert message provides the user with real-time updates on the eye health status in form of the generated feedback, and ensuring prompt intervention if needed.

Optionally, the method further comprises:

determining if a position of the optical apparatus with respect to the user's eye is inappropriate for measuring the temperature of the ocular surface of the user's eye; and
when it is determined that the position of the optical apparatus with respect to the user's eye is inappropriate:

measuring a temperature of skin around the user's eye, using at least one another temperature sensor in the optical apparatus, at the first plurality of time instants within the first time duration and the second plurality of time instants within the second time duration, respectively, and
adjusting the measured temperature of the ocular surface of the user's eye at the first time plurality of time instants and the second plurality of time instants, based on the measured temperature of the skin around the ocular surface of the user's eye at the first plurality of time instants and the second plurality of time instants, respectively.

In this regard, the term *"position"* refers to a spatial arrangement or orientation of the optical apparatus on the user's face relative to the user's eye. Notably, the position of the optical apparatus ensures the at least one temperature sensor is correctly aligned and placed at an appropriate distance to accurately measure the temperature of the ocular surface of the user's eye. Optionally, the optical apparatus may be equipped with sensors (for example, eye trackers) to detect the position of the optical apparatus relative to the user's eye. Herein, the term *"another temperature sensor"* refers to a skin temperature sensor that is used to measure the temperature of the skin around the user's eye. Notably, the at least one another temperature sensor measures the temperature of skin around the user's eye at the first plurality of time instants within the first time duration and at the second plurality of time instants within the second time duration, respectively. Optionally, a difference in the temperature of the skin around the user's eye during the first time duration and the second time duration is indicative of the position of the optical apparatus with respect to the user's eye to be inappropriate. The difference in the temperature of the skin of the user's face may indicate that the optical apparatus is pulled away from the face, and due to increased distance between the frame and the user's skin, radiated temperature from the user's skin decreases and sensor provides incorrect data regarding the temperature of the user's skin. Beneficially, measuring the temperature of the skin around the user's eye ensures that effects of the temperature of the skin around the user's eye on the measured temperature of the ocular surface of the user's eye is effectively negated. Notably, the temperature of the skin around the user's eye facilitates to accurately measure the temperature of the ocular surface of user's eye which is difficult due to the inappropriate position of the optical apparatus with respect to the user's eye.

Moreover, after measuring the temperature of the skin around the user's eye, the at least one processor calibrate or adjust the measured temperature of the ocular surface of the user's eye using the measured temperature of the skin around the user's eye. Notably the measured temperature of the ocular surface of the user's eye at the first plurality of time instants and the second plurality of time instants, are adjusted, respectively. A technical effect is that the precision of measuring the temperature of the ocular surface of the user's eye is significantly enhanced, even in scenarios when the position of the optical apparatus with respect to the user's eye is inappropriate. Additionally, the at least one processor detects the changes in the temperature of the user's during the first time duration and the second time duration and discards the said changes, as the result of movement of the optical apparatus, from the analysis to provide more accurate results.

The present disclosure also relates to the optical apparatus as described above. Various embodiments and variants disclosed above, with respect to the aforementioned method, apply mutatis mutandis to the optical apparatus.

Notably, the frame is employed to hold at least one optical element (for example, an optical lens) per eye of the user. It will be appreciated that a material of the frame could be plastic, metal, polymer, and the like. Moreover, the frame may be lightweight, ergonomically designed, and easy to use. It will be appreciated that the optical apparatus is in use when the optical apparatus is worn on the user's head. Optionally, the at least one temperature sensor is arranged on one of: a rim, a bridge, of the frame, and the like.

Optionally, the at least one processor is coupled to the at least one temperature sensor in a wired or wireless manner. Notably, prior to estimating the temperature of the ocular surface of the user's eye, the at least one processor is configured to receive sensor data from the at least one temperature sensor, the sensor data being indicative of the measured temperature of the ocular surface of the user's eye.

Optionally, to determine the change in temperature trend, the at least one processor is further configured to determine one of:

a change in slope between a first slope of the first temperature trend and a second slope of the second temperature trend,

a change in average temperature between a first average temperature of the first temperature trend and a second average temperature of the second temperature trend,

a change in initial temperature between a first initial temperature in the first temperature trend and a second initial temperature in the second temperature trend, or

a change in given temperature between a first given temperature amongst the first temperature trend at a first given time instant amongst the first duration and a second given temperature amongst the second temperature trend at a second given time instant amongst the second time duration.

Optionally, the at least one processor is further configured to store data related to the deemed use of the eye drops in the user's eye during the predefined time period of the optical apparatus not being in use in a database.

Optionally, the at least one processor further configured to:

generate a feedback for the user, based on the determined change in temperature trend; and send the feedback as an alert message to a user device communicably coupled to the at least one processor.

Optionally, a sensing area of the at least one temperature sensor is aligned with a horizontal axis passing through the user's eye, and wherein the at least one temperature sensor is arranged on one of: a nasal side, a hinge side of the optical apparatus.

In this regard, the term *"nasal side"* refers to an inner side of the frame of the optical apparatus that is closer to the user's nose. Notably, arranging the at least one temperature sensor on the nasal side of the optical apparatus allows for proximity to the medial part of the eye, which is closer to tear duct. The area may show significant temperature changes when the eye drops are applied. The term *"hinge side"* refers to outer side of the frame of the optical apparatus where the frame connect to temples (arm of the optical apparatus) via hinges. Notably, arranging the at least one temperature sensor on the hinge side provides coverage for lateral part of the eye. Moreover, the hinge side facilitates to capture temperature changes across a different region of the ocular surface. A technical effect of the specific arrangement of the at least one temperature sensor on either the nasal side or the hinge side of the optical apparatus to provide comprehensive and accurate temperature measurement. Moreover, alignment of the sensing area with the horizontal axis passing through the user's eye helps in maintaining consistent positioning relative to the ocular surface.

Optionally, the at least one processor is configured to:

determine if a position of the optical apparatus with respect to the user's eye is inappropriate to measure the temperature of the ocular surface of the user's eye; and
when it is determined that the position of the optical apparatus with respect to the user's eye is inappropriate:

measure a temperature of skin around the user's eye, using at least one another temperature sensor in the optical apparatus, at the first plurality of time instants within the first time duration and the second plurality of time instants within the second time duration, respectively, and adjust the measured temperature of the ocular surface of the user's eye at the first plurality of time instants and the second plurality of time instants, based on the measured temperature of the skin around the ocular surface of the user's eye at the first plurality of time instants and the second plurality of time instants, respectively.

DETAILED DESCRIPTION OF THE DRAWINGS

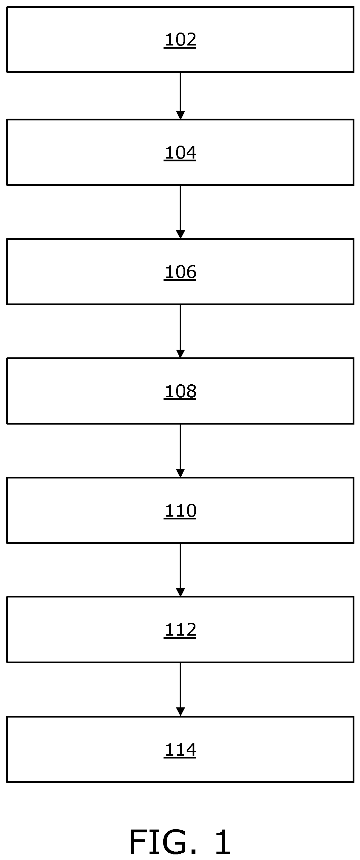

Referring to FIG. 1, illustrated is a flowchart for depicting steps of a method for monitoring a use of eye drops in a user's eye, in accordance with an embodiment of the present disclosure. At step **102,** a temperature of an ocular surface of the user's eye is measured, using at least one temperature sensor in an optical apparatus worn by a user, at a first plurality of time instants within a first time duration, prior to the optical apparatus not being in use for a predefined time period. At step **104,** a first temperature trend of the ocular surface of the user's eye is generated, for the first time duration, based on the measured temperature at the first plurality of time instants within the first time duration, using at least one processor in the optical apparatus communicably coupled to the at least one temperature sensor. At step **106,** the temperature of the ocular surface of the user's eye is measured, using the at least one temperature sensor, at a second plurality of time instants within a second time duration, subsequent to the optical apparatus being in use, wherein the optical apparatus is brought to use after the optical apparatus not being in use for the predefined time period. At step **108,** a second temperature trend of the ocular surface of the user's eye is generated, for the second time duration, based on the measured temperature at the second plurality of time instants within the second time duration, using the at least one processor. At step **110,** a change in temperature trend is determined between the first temperature trend and the second temperature trend of the ocular surface of the user's eye, using the at least one processor. At step **112,** if the change in temperature trend is greater than a predefined value is determined, using the at least one processor. At step **114,** when it is determined that the change in temperature trend is greater than the predefined value, the use of the eye drops in the user's eyes is deemed during the predefined time period of the optical apparatus not being in use.

The aforementioned steps are only illustrative and other alternatives can also be provided where one or more steps are added, one or more steps are removed, or one or more steps are provided in a different sequence without departing from the scope of the claims herein.

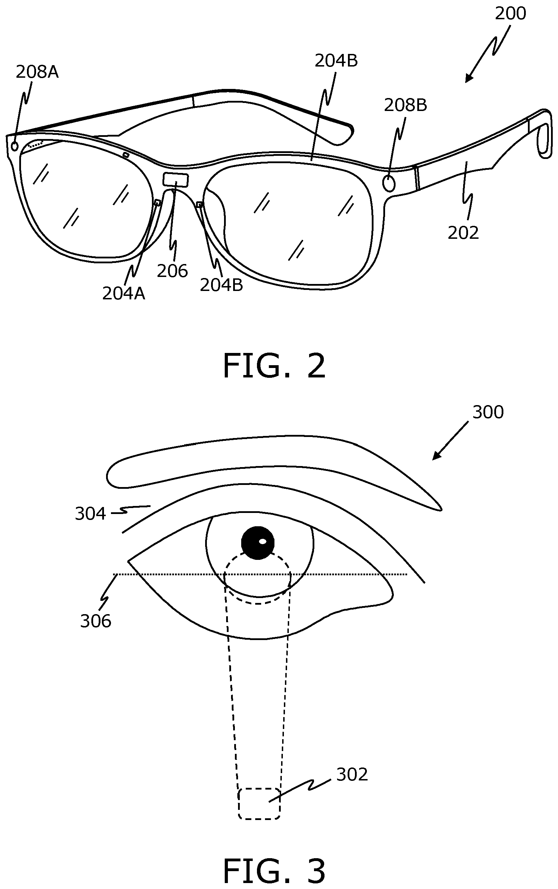

Referring to FIG. 2, illustrated is a schematic illustration of an optical apparatus **200,** in accordance with an embodiment of the present disclosure. As shown, the optical apparatus **200** comprises a frame **202.** Moreover, the optical apparatus **200** comprises at least one temperature sensor (depicted as a first temperature sensor **204A,** and a second temperature sensor **204B**) arranged on the frame **202,** wherein when the optical apparatus **200** is in use, the at least one temperature sensor **204A** and **204B** faces a user's eye. Furthermore, the optical apparatus **200** comprises at least one processor (depicted as a processor **206**) coupled to the at least one temperature sensor **204A** and **204B,** wherein the at least one processor **206** is configured to control the at least one temperature sensor **204A** and **204B** to measure a temperature of an ocular surface of the user's eye, at a first plurality of time instants within a first time duration, prior to the optical apparatus not being in use for a predefined time period. Moreover, the at least one processor **206** is configured to generate a first temperature trend of the ocular surface of the user's eye, for the first time duration, based on the measured temperature at the first plurality of time instants within the first time duration. Furthermore, the at least one processor **206** is configured to control the at least one temperature sensor **204A** and **204B** to measure the temperature of the ocular surface of the user's eye, at a second plurality of time instants within a second time duration, subsequent to the optical apparatus **200** being in use, wherein the optical apparatus **200** is brought to use after the optical apparatus **200** not being in use for the predefined time period. Furthermore, the at least one processor **206** is configured to generate a second temperature trend of the ocular surface of the user's eye, for the second time duration, based on the measured temperature at the second plurality of time instants within the second time duration. Furthermore, the at least one processor **206** is configured to determine a change in temperature trend between the first temperature trend and the second temperature trend of the ocular surface of the user's eye. Furthermore, the at least one processor **206** is configured to determine if the change in temperature trend is greater than a predefined value and when it is determined that the change in temperature trend is greater than the predefined value, deem the use of the eye drops in the user's eyes during the time period of the optical apparatus **200** not being in use. Optionally, the optical apparatus **200** further comprises at least one another temperature sensor (depicted as a first another temperature sensor **208A,** and a second another temperature sensor **208B**) to measure a temperature of skin around the user's eye.

FIG. 2 is merely an example, which should not unduly limit the scope of the claims herein. It is to be understood that the specific implementations of the optical apparatus **200** are provided as examples and are not to be construed as limiting it to specific numbers, sizes, or shapes of the at least one temperature sensor **204A** and **204B,** the at least one processor **206,** the at least one another temperature sensor **208A** and **208B,** and similar. A person skilled in the art will recognize many variations, alternatives, and modifications of embodiments of the present disclosure.

Referring to FIG. 3, illustrated is a schematic illustration of a user's eye **300** when an optical apparatus is in use, in accordance with an embodiment of the present disclosure. As shown, the temperature of an ocular surface of the user's eye **300** is measured, using at least one temperature sensor (depicted as a first temperature sensor **302**). Moreover, a sensing area **304** of at least one temperature sensor **302** is aligned with a horizontal axis **306** passing through the user's eye **300.**

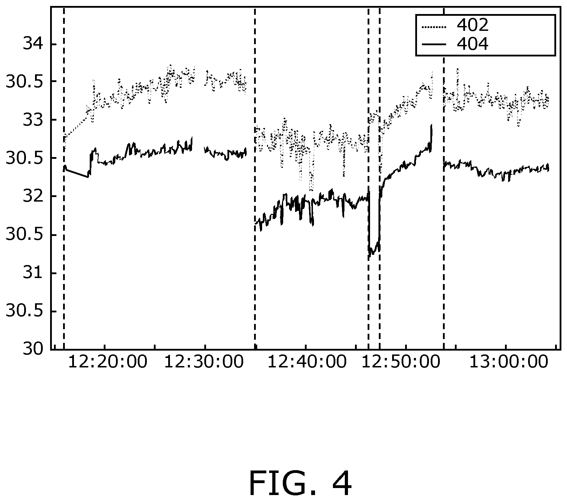

Referring to FIG. 4, illustrated is a graphical representation **400** depicting a first temperature trend **402** and a second temperature trend **404,** in accordance with an embodiment of the present disclosure. As shown, the x-axis of the graphical representation **400** depicts a period of 60 minutes for the corresponding day, and the y-axis of the graphical representation **400** depicts a temperature of the ocular surface of the user's eye.

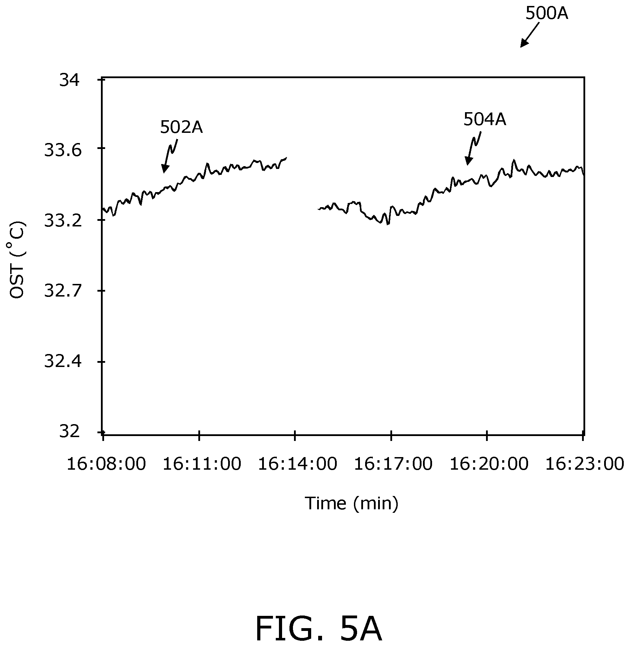

Referring to FIGs. 5A-C, illustrated are graphical representations **500A-C** depicting a first temperature trend **504A-C** and a second temperature trend **504A-D** for the first time duration and the second time duration, respectively, in accordance with an embodiment of the present disclosure. As shown, the x-axis of the graphical representations **500A-C** depicts a period of 60 minutes, and the y-axis of the graphical representations **500A-C** depicts a temperature of the ocular surface of the user's eye. As shown in FIG. 5A, the slope of the first temperature trend **502A** is positive and steep. However, the slope of second temperature trend **504A** is less steep than the first temperature trend **502A,** when a type of the eye drops is lipid-free. Subsequently, after the lipid-free eye drops evaporate, the temperature of the ocular surface of the user's eye rises back to original temperature before the use of the lipid-free eye drops and the slope of the second temperature trend **504A** becomes steeper and positive.

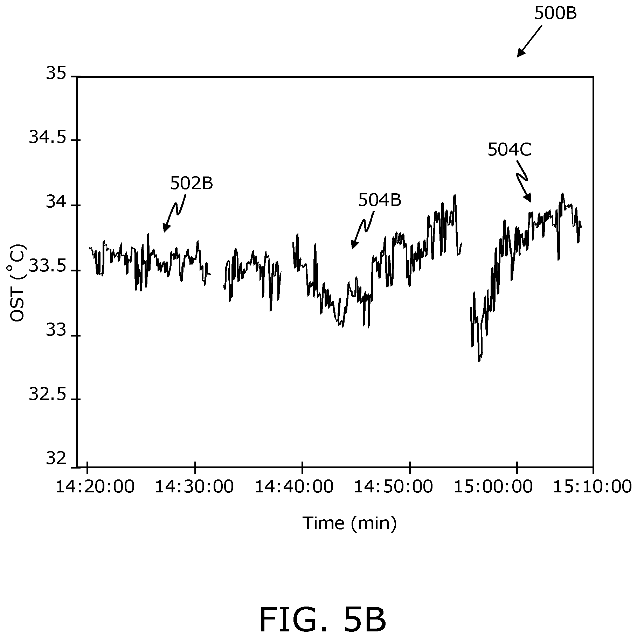

As shown in FIG. 5B, the slope of the first temperature trend **502B** is flat, signifying nearly a constant value for the temperature of the ocular surface of the user's eye. However, in the second temperature trend **504B,** there is a small increase in the temperature of the ocular surface of the user's eye, when the type of the eye drops is lipid-based. Subsequently, there is strong increase in the temperature of the ocular surface of the user's eye due to limited evaporation of lipid layer in the user's eye. However, the slope of the second temperature trend **504C** may be steep and the temperature of the ocular surface of the user's eye returns back to initial value, when the type of the eye drops is lipid-free.

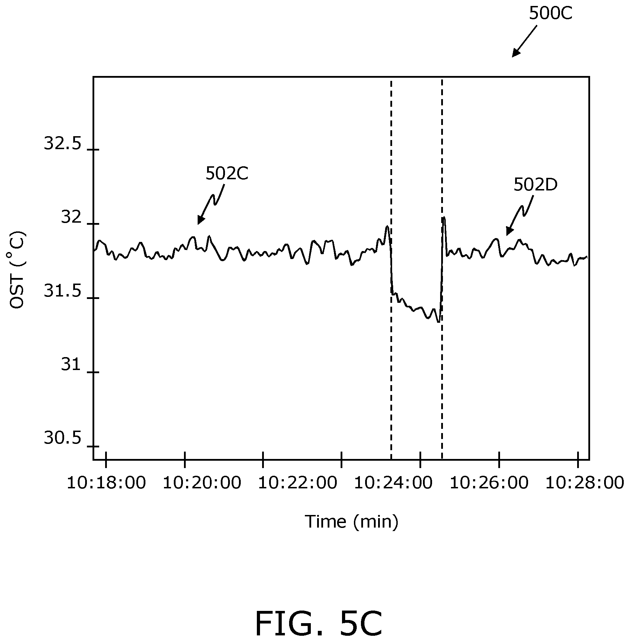

As shown in FIG. 5C, the first temperature trend **502C** of the ocular surface of the user's eye decreases steeply and when the effect of the eye drops decreases, thereafter there is increase in the temperature of the ocular surface of the user's eye to the initial value in the second temperature trend **502D.** However, it will be appreciated that the steep change in the temperature trend due to the movement of the optical apparatus can be detected by the at least one processor and discarded by the at least one processor from the analysis of the temperature trend.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

**Claims**

1. A method for monitoring a use of eye drops in a user's eye (300), wherein the method comprising:

    measuring a temperature of an ocular surface of the user's eye, using at least one temperature sensor in an optical apparatus worn by a user, at a first plurality of time instants within a first time duration, prior to the optical apparatus (200) not being in use for a predefined time period, wherein the first time duration corresponds to a period when the optical apparatus is worn by the user before the optical apparatus is taken off;
    wherein the at least one temperature sensor is at least one thermopile IR sensor configured to measure infrared emission between a range of 8 to 14 micrometers;
    generating a first temperature trend of the ocular surface of the user's eye, for the first time duration, based on the measured temperature at the first plurality of time instants within the first time duration, using at least one processor (206) in the optical apparatus communicably coupled to the at least one temperature sensor (204A, 204B, 302),
    wherein a normal ocular temperature of the user's eye is in a range of 32°C and 36°C;

    wherein the predefined time period of the optical apparatus not being in use is in a range of 30 seconds to 5 minutes, and wherein the first time duration and the second time duration are in a range of 5 minutes to 15 minutes;
    measuring the temperature of the ocular surface of the user's eye, using the at least one temperature sensor, at a second plurality of time instants within a second time duration, subsequent to the optical apparatus being in use, wherein the optical apparatus is brought to use after the optical apparatus not being in use for the predefined time period, wherein the optical apparatus is brought to use after the optical apparatus not being in use for the predefined time period, wherein the second time duration corresponds to a period when the optical apparatus is worn by the user after the optical apparatus is put back on;
    generating a second temperature trend of the ocular surface of the user's eye, for the second time duration, based on the measured temperature at the second plurality of time instants within the second time duration, using the at least one processor;
    determining a change in temperature trend between the first temperature trend and the second temperature trend of the ocular surface of the user's eye, using the at least one processor;
    determining if the change in temperature trend is greater than a predefined value, using the at least one processor; and
    when it is determined that the change in temperature trend is greater than the predefined value, deeming the use of the eye drops in the user's eyes during the predefined time period of the optical apparatus not being in use.

2. A method according to claim 1, wherein the step of determining the change in temperature trend comprises determining one of:

    a change in slope between a first slope of the first temperature trend and a second slope of the second temperature trend,
    a change in average temperature between a first average temperature of the first temperature trend and a second average temperature of the second temperature trend,
    a change in initial temperature between a first initial temperature in the first temperature trend and a second initial temperature in the second temperature trend, or
    a change in given temperature between a first given temperature amongst the first temperature trend at a first given time instant amongst the first duration and a second given temperature amongst the second temperature trend at a second given time instant amongst the second time duration.

3. A method according to claim 1 or 2, further comprising storing data related to the deemed use of the eye drops in the user's eye (300) during the predefined time period of the optical apparatus (200) not being in use in a database.

4. A method according to any of the preceding claims, further comprising:

generating a feedback for the user, based on the determined change in temperature trend, using the at least one processor (206); and
sending the feedback as an alert message to a user device communicably coupled to the at least one processor.

5. A method according to any of the preceding claims, wherein the predefined time period of the optical apparatus (200) not being in use is in a range of 30 seconds to 5 minutes, and wherein the first time duration and the second time duration are in a range of 5 minutes to 15 minutes.

6. A method according to any of the preceding claims, wherein the predefined value is adjusted based on a type of the eye drops.

7. A method according to any of the preceding claims, wherein the at least one temperature (204A, 204B, 302) is at least one thermopile IR sensor.

8. A method according to any of the preceding claims, wherein a sensing area (304) of the at least one temperature sensor (204A, 204B, 302) is aligned with a horizontal axis (306) passing through the user's eye (300).

9. A method according to any of the preceding claims, further comprising:

determining if a position of the optical apparatus (200) with respect to the user's eye (300) is inappropriate for measuring the temperature of the ocular surface of the user's eye; and
when it is determined that the position of the optical apparatus with respect to the user's eye is inappropriate:

measuring a temperature of skin around the user's eye, using at least one another temperature sensor (208A, 208B) in the optical apparatus, at the first plurality of time instants within the first time duration and the second plurality of time instants within the second time duration, respectively, and

adjusting the measured temperature of the ocular surface of the user's eye at the first time plurality of time instants and the second plurality of time instants, based on the measured temperature of the skin around the ocular surface of the user's eye at the first plurality of time instants and the second plurality of time instants, respectively.

10. An optical apparatus comprising:
a frame (202);

at least one temperature sensor (204A, 204B, 302) arranged on the frame, wherein when the optical apparatus is in use, the at least one temperature sensor faces a user's eye (300), wherein the at least one temperature sensor is at least one thermopile IR sensor configured to measure infrared emission between a range of 8 to 14 micrometers; and
at least one processor (206) coupled to the at least one temperature sensor, wherein the at least one processor is configured to:

control the at least one temperature sensor to measure a temperature of an ocular surface of the user's eye, at a first plurality of time instants within a first time duration, prior to the optical apparatus not being in use for a predefined time period, wherein the first time duration corresponds to a period when the optical apparatus is worn by the user before the optical apparatus is taken off;
generate a first temperature trend of the ocular surface of the user's eye, for the first time duration, based on the measured temperature at the first plurality of time instants within the first time duration, wherein a normal ocular temperature of the user's eye is in a range of 32°C and 36°C;
wherein the predefined time period of the optical apparatus not being in use is in a range of 30 seconds to 5 minutes, and wherein the first time duration and the second time duration are in a range of 5 minutes to 15 minutes;
control the at least one temperature sensor to measure the temperature of the ocular surface of the user's eye, at a second plurality of time instants within a second time duration, subsequent to the optical apparatus being in use, wherein the optical apparatus is brought to use after the optical apparatus not being in use for the predefined time period;
generate a second temperature trend of the ocular surface of the user's eye, for the second time duration, based on the measured temperature at the second plurality of time instants within the second time duration, wherein the second time duration corresponds to a period when the optical apparatus is worn by the user after the optical apparatus is put back on;

determine a change in temperature trend between the first temperature trend and the second temperature trend of the ocular surface of the user's eye;

determine if the change in temperature trend is greater than a predefined value; and when it is determined that the change in temperature trend is greater than the predefined value, deem the use of the eye drops in the user's eyes during the time period of the optical apparatus not being in use.

11. An optical apparatus according to claim 10, wherein to determine the change in temperature trend, the at least one processor (206) is further configured to determine one of:

a change in slope between a first slope of the first temperature trend and a second slope of the second temperature trend,

a change in average temperature between a first average temperature of the first temperature trend and a second average temperature of the second temperature trend,

a change in initial temperature between a first initial temperature in the first temperature trend and a second initial temperature in the second temperature trend, or

a change in given temperature between a first given temperature amongst the first temperature trend at a first given time instant amongst the first duration and a second given temperature amongst the second temperature trend at a second given time instant amongst the second time duration.

12. An optical apparatus according to claim 10 or 11, wherein the at least one processor (206) is further configured to store data related to the deemed use of the eye drops in the user's eye during the predefined time period of the optical apparatus (200) not being in use in a database.

13. An optical apparatus according to any of the claims 10-12, the at least one processor (206) further configured to:

generate a feedback for the user, based on the determined change in temperature trend; and send the feedback as an alert message to a user device communicably coupled to the at least one processor.

14. An optical apparatus according to any of the claims 10-13, wherein a sensing area (304) of the at least one temperature sensor (304, 204A, 204B) is aligned with a horizontal axis (306) passing through the user's eye (300), and wherein the at least one temperature sensor is arranged on one of: a nasal side, a hinge side of the optical apparatus (200).

15. An optical apparatus according to any of the preceding claims, wherein the at least one processor (206) is configured to:

determine if a position of the optical apparatus (200) with respect to the user's eye (300) is inappropriate to measure the temperature of the ocular surface of the user's eye; and when it is determined that the position of the optical apparatus with respect to the user's eye is inappropriate:

measure a temperature of skin around the user's eye, using at least one another temperature sensor (208A, 208B) in the optical apparatus, at the first plurality of time instants within the first time duration and the second plurality of time instants within the second time duration, respectively, and adjust the measured temperature of the ocular surface of the user's eye at the first plurality of time instants and the second plurality of time instants, based on the measured temperature of the skin around the ocular surface of the user's eye at the first plurality of time instants and the second plurality of time instants, respectively.

FIG. 1

FIG. 2

FIG. 3

FIG. 4

FIG. 5A

FIG. 5B

FIG. 5C

# EUROPEAN SEARCH REPORT

**Application Number**

EP 25 19 8740

## DOCUMENTS CONSIDERED TO BE RELEVANT

| Category | Citation of document with indication, where appropriate, of relevant passages | Relevant to claim |
|---|---|---|
| Y | WO 2018/209289 A1 (UNIV CALIFORNIA [US]) 15 November 2018 (2018-11-15) * paragraphs [0033] - [0070]; figures 1-6 * | 1-15 |
| Y | US 2021/393139 A1 (MANNESCHI ALESSANDRO [IT] ET AL) 23 December 2021 (2021-12-23) * paragraph [0111] * | 1-15 |
| Y | YVONNE WILLIAMS: "Intrusion Detection", IEEE DRAFT WGDS; 914955072758, IEEE-SA IMEET CENTRAL, PISCATAWAY, NJ USA , vol. NPEC WG 3.2 2 February 2022 (2022-02-02), pages 1-208, XP068268823, Retrieved from the Internet: URL:https://ieee-sa.imeetcentral.com/p/aQA AAAAE4_Zi [retrieved on 2022-02-05] * pages 4-16, paragraph 2. - paragraph 3. * | 1-15 |
| Y | US 2022/023334 A1 (COOPER EUGENE REX [US] ET AL) 27 January 2022 (2022-01-27) * paragraph [0113] * | 6 |
| Y | WO 2011/129461 A1 (R TECH UENO LTD [JP]; MASHIMA YUKIHIKO [JP]; UENO RYUJI [US]) 20 October 2011 (2011-10-20) * paragraph [0141] * | 8,14 |
| Y | US 2019/151142 A1 (KRECK THOMAS [US] ET AL) 23 May 2019 (2019-05-23) * paragraph [0401] * | 9,15 |

**CLASSIFICATION OF THE APPLICATION (IPC)**

INV.
A61B3/10

ADD.
A61B5/00

**TECHNICAL FIELDS SEARCHED (IPC)**

A61B

The present search report has been drawn up for all claims

| Place of search | Date of completion of the search | Examiner |
|---|---|---|
| Munich | 9 January 2026 | Mäki-Mantila, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 8740

This annex lists the patent family members relating to the patent documents cited in  the above-mentioned European search report.
The members are as contained in the European Patent Office EDP file on
The European Patent Office is in no way liable for these particulars which are merely given for the purpose of information.

09-01-2026

| Patent document cited in search report | | | Publication date | Patent family member(s) | | Publication date |
|---|---|---|---|---|---|---|
| WO 2018209289 | A1 | 15-11-2018 | US | 2020138367 A1 | | 07-05-2020 |
| | | | WO | 2018209289 A1 | | 15-11-2018 |
| US 2021393139 | A1 | 23-12-2021 | EP | 3929549 A1 | | 29-12-2021 |
| | | | FR | 3111700 A1 | | 24-12-2021 |
| | | | US | 2021393139 A1 | | 23-12-2021 |
| US 2022023334 | A1 | 27-01-2022 | AU | 2016413933 A1 | | 11-04-2019 |
| | | | CA | 3029391 A1 | | 11-01-2018 |
| | | | MX | 395496 B | | 25-03-2025 |
| | | | US | 2019134079 A1 | | 09-05-2019 |
| | | | US | 2022023334 A1 | | 27-01-2022 |
| | | | WO | 2018009177 A1 | | 11-01-2018 |
| WO 2011129461 | A1 | 20-10-2011 | AR | 080888 A1 | | 16-05-2012 |
| | | | CA | 2795720 A1 | | 20-10-2011 |
| | | | CA | 2795723 A1 | | 20-10-2011 |
| | | | CN | 102933217 A | | 13-02-2013 |
| | | | CN | 102946883 A | | 27-02-2013 |
| | | | EP | 2558103 A1 | | 20-02-2013 |
| | | | EP | 2558104 A1 | | 20-02-2013 |
| | | | JP | 5686819 B2 | | 18-03-2015 |
| | | | JP | 5878128 B2 | | 08-03-2016 |
| | | | JP | 2013523601 A | | 17-06-2013 |
| | | | JP | 2013528563 A | | 11-07-2013 |
| | | | JP | 2016026182 A | | 12-02-2016 |
| | | | KR | 20130050939 A | | 16-05-2013 |
| | | | KR | 20130099812 A | | 06-09-2013 |
| | | | TW | 201141486 A | | 01-12-2011 |
| | | | TW | 201204366 A | | 01-02-2012 |
| | | | US | 2011275711 A1 | | 10-11-2011 |
| | | | US | 2011275715 A1 | | 10-11-2011 |
| | | | WO | 2011129457 A1 | | 20-10-2011 |
| | | | WO | 2011129461 A1 | | 20-10-2011 |
| US 2019151142 | A1 | 23-05-2019 | AU | 2012286892 A1 | | 02-05-2013 |
| | | | CA | 2841863 A1 | | 31-01-2013 |
| | | | CN | 103826689 A | | 28-05-2014 |
| | | | EP | 2736581 A2 | | 04-06-2014 |
| | | | JP | 2014529315 A | | 06-11-2014 |
| | | | KR | 20140048305 A | | 23-04-2014 |
| | | | US | 2013030411 A1 | | 31-01-2013 |
| | | | US | 2016296365 A1 | | 13-10-2016 |
| | | | US | 2019151142 A1 | | 23-05-2019 |
| | | | WO | 2013016437 A2 | | 31-01-2013 |

EPO FORM P0459

For more details about this annex : see Official Journal of the European Patent Office, No. 12/82